(19) **Europäisches Patentamt / European Patent Office / Office européen des brevets**

(11) **EP 4 596 761 A1**

(12) **EUROPEAN PATENT APPLICATION**

(43) Date of publication:
06.08.2025 Bulletin 2025/32

(21) Application number: 24154622.5

(22) Date of filing: 30.01.2024

(51) International Patent Classification (IPC):
$C25D\ 5/36^{(2006.01)}$     $C25D\ 5/50^{(2006.01)}$
$C23C\ 14/16^{(2006.01)}$    $C23C\ 14/58^{(2006.01)}$
$C23C\ 16/06^{(2006.01)}$    $C25D\ 3/38^{(2006.01)}$

(52) Cooperative Patent Classification (CPC):
C25D 5/50; C23C 14/16; C23C 14/5806;
C23C 16/06; C25D 5/36; C25D 3/38

(84) Designated Contracting States:
AL AT BE BG CH CY CZ DE DK EE ES FI FR GB
GR HR HU IE IS IT LI LT LU LV MC ME MK MT NL
NO PL PT RO RS SE SI SK SM TR
Designated Extension States:
BA
Designated Validation States:
GE KH MA MD TN

(71) Applicant: Tung Mung Development Co., Ltd.
Tainan City 726 (TW)

(72) Inventors:
• Tsai, Wen-Ta
701037 Tainan City (TW)
• Liu, Bernard Haochih
701037 Tainan City (TW)
• Chen, Zhi-Yan
427004 Taichung City (TW)
• Huang, Chong Cheng
710026 Tainan City (TW)

(74) Representative: Jannig & Repkow
Patentanwälte PartG mbB
Klausenberg 20
86199 Augsburg (DE)

(54) **METHOD FOR PREPARING ANTIBACTERIAL STAINLESS STEEL BY SURFACE ALLOYING**

(57)     A method for preparing antibacterial stainless steel by surface alloying includes the steps of coating an infiltration promoter layer on a stainless steel surface, coating an antibacterial metal layer on a surface of the infiltration promoter layer, and performing heat treatment of the stainless steel to diffuse an antibacterial metal into the stainless steel. This method can be applied to various types of stainless steel, and the antibacterial metal can be diffused and quenched into the stainless steel, such that the finally formed surface of the stainless steel has an antibacterial alloy layer with a specific thickness to provide better corrosion resistance and antibacterial ability without changing the advantages and properties of the antibacterial metal or stainless steel substrate, and the thickness and concentration of the antibacterial metal layer, and the parameters for heat treatment can be adjusted to control the chemical composition and thickness of the antibacterial alloy layer.

Coat an infiltration promoter layer on a stainless steel surface. — S001

Coat an antibacterial metal layer. — S002

Perform a heat treatment of the stainless steel. — S003

FIG.1

EP 4 596 761 A1

## Description

## BACKGROUND

**Technical Field**

The present disclosure relates to a method for preparing antibacterial stainless steel by surface alloying, and more particularly to the method of coating an antibacterial metal layer on a stainless steel surface and performing heat treatment to diffuse an antibacterial metal into the stainless steel surface in order to form an antibacterial alloy layer with a specific thickness and achieve the effect of good corrosion resistance and antibacterial ability.

**Description of Related Art**

Stainless steel has the advantages of good appearance and high corrosion resistance, and it is thus widely used in household products, kitchenware, medical equipment, etc. However, with the rise of hygiene and safety awareness, manufacturers pay increasingly more attention on the antibacterial function in the development of stainless steel products. The present antibacterial technology for stainless steel is mainly divided into two types: (1) surface coating type and (2) alloy type. The former coats a substance with the antibacterial function on the stainless steel surface by various different methods, and its antibacterial effect is related to the adhesiveness and thickness of the coated layer. After the coated layer is worn out, the antibacterial effect will no longer exist. The latter adds an alloy element with the antibacterial function into the stainless steel substrate, and its antibacterial effect is closely related to the content, phase, composition and distribution of the alloy element with the antibacterial function. Since the surface coating type stainless steel generally has the issues of easily peeled-off antibacterial coating and poor wear resistance, and it is easy to lose the antibacterial function when the antibacterial layer is worn out, therefore the present antibacterial stainless steel is mainly alloy type stainless steel.

The common antibacterial elements used for the alloy type antibacterial stainless steel are mainly silver and copper, and these antibacterial metal elements can be released from the stainless steel surface in the form of metal ions to provide the bacteriostatic and antibacterial effects, and the antibacterial effect is related to the concentration of these antibacterial metal elements in the stainless steel. The present alloy type antibacterial stainless steel products are mainly manufactured by a fusing method. After the antibacterial element and the stainless steel are mixed and molten at high temperature, the antibacterial element is molten into the substrate. The feature of using this manufacturing process is that the antibacterial element will be distributed uniformly on the substrate, such that the entire stainless steel has the antibacterial function. However, the antibacterial reaction process only takes place at the stainless steel surface. In most cases, the whole piece of stainless steel material does not need to contain the antibacterial element. These additionally added antibacterial elements not only will increase the production cost, but also will cause changes in the performance of the substrate.

In a conventional method of processing the stainless steel surface, the antibacterial alloy layer is prepared by the ion implantation and shot peening. Although such manufacturing process can precisely control the concentration of the antibacterial element, the diffusion depth is too narrow, and it is difficult to guarantee the antibacterial life, and the manufacturing cost is very high and unfavourable to mass production.

Related art further provides a "stainless steel material with antibacterial and antiviral properties and a manufacturing method thereof' as disclosed in the R.O.C. Pat. No. 1764530, wherein a copper layer is diffused into a steel surface by heat treatment to form an antibacterial alloy layer. Although the manufacturing process using this method has more production advantages, the diffusion depth of the antibacterial diffusion layer is limited, which fails to meet the manufacturing requirements for specific products and applications. Furthermore, although this patent states that its method is applicable for all types of stainless steel, test results on the data of actual experiments show that it is difficult to produce the copper diffusion effect on ferritic stainless steel and martensitic stainless steel (such as SS440C the stainless steel), and it is found that there is almost no mutual diffusion between the substrate and the copper layer, so that this method may not be fully mature.

In view of the aforementioned problems of the related art, the present discloser conducted extensive research and experiment on the surface antibacterial treatment of the stainless steel in hope of providing a better solution to solve the aforementioned problems, and finally invented the method in accordance with this disclosure to overcome the problems of the related art.

## SUMMARY

Therefore, it is a primary objective of the present disclosure to provide a method for preparing antibacterial stainless steel by surface alloying, and the method includes the steps of: coating an infiltration promoter layer on a stainless

EP 4 596 761 A1

steel surface; coating an antibacterial metal layer on a surface of the infiltration promoter layer; and performing heat treatment of the stainless steel, such that the antibacterial metal is diffused into the stainless steel.

In the method for preparing antibacterial stainless steel by surface alloying in accordance with this disclosure, the stainless steel is at least one selected from ferritic stainless steel, austenitic stainless steel, martensitic stainless steel, or duplex stainless steel.

In the method for preparing antibacterial stainless steel by surface alloying in accordance with this disclosure, the antibacterial metal layer is coated on the stainless steel surface by a method selected from physical vapor deposition, chemical vapor deposition, electroplating or autocatalytic plating.

In the method for preparing antibacterial stainless steel by surface alloying in accordance with this disclosure, the antibacterial metal layer is coated when the thickness of the stainless steel surface falls within a range from $0.1\mu m$ to $15\mu m$.

In the method for preparing antibacterial stainless steel by surface alloying in accordance with this disclosure, the heat treatment heats in the protective atmosphere to a temperature above 600°C, and the heating time is greater than 1 minute.

The method for preparing antibacterial stainless steel by surface alloying in accordance with this disclosure further includes the step of performing a cooling treatment of the stainless steel having the antibacterial metal layer by furnace cooling, air cooling) or quench.

In the method for preparing antibacterial stainless steel by surface alloying in accordance with this disclosure, the cooling treatment cools the stainless steel having the antibacterial metal layer to room temperature.

In the method for preparing antibacterial stainless steel by surface alloying in accordance with this disclosure, the antibacterial metal is copper.

In the method for preparing antibacterial stainless steel by surface alloying in accordance with this disclosure, the infiltration promoter layer is silver, cobalt, nickel, zinc, or molybdenum, or any combination of the above.

In the method for preparing antibacterial stainless steel by surface alloying in accordance with this disclosure, the infiltration promoter element has content less than the content of the antibacterial metal.

In view of the description above, this disclosure has the following advantages and effects:

The manufacturing process of this disclosure can be applied for the manufacture of various types of stainless steel. In the process of this disclosure, the antibacterial metal is diffused and quenched into the stainless steel by coating the antibacterial metal layer and performing heat treatment, such that the finally formed stainless steel surface has the antibacterial alloy layer with a specific thickness to provide better corrosion resistance and antibacterial ability without changing the advantages and properties of the antibacterial metal or stainless steel substrate, and the thickness of the antibacterial metal layer can be increased effectively by adding the infiltration promoter layer, especially for the ferritic and martensitic stainless steel. In addition, the thickness and concentration of the antibacterial metal layer, and the parameters for heat treatment can be adjusted to control the chemical composition and thickness of the antibacterial alloy layer. Therefore, this method has a wide range of applicability. In addition, the processing method of this disclosure is simple, easy and fast without requiring the use of large boilers and furnaces (as required for the preparing of the conventional alloy type antibacterial stainless steel), and thus the method of this disclosure can significantly reduce the level of difficulty and cost of the antibacterial process of the stainless steel and effectively improve production capacity and efficiency.

## BRIEF DESCRIPTION OF THE DRAWINGS

FIG. 1 is a flow chart of this disclosure;
FIG. 2a is a cross-section SEM image of Specimen No. 1 of this disclosure after coating an antibacterial metal layer and before taking heat treatment;
FIG. 2b is a cross-section SEM image of Specimen No. 1 of this disclosure after taking heat treatment;
FIG. 2c is a cross-section SEM image in a partial blow-up view of the surface of Specimen No. 1 of this disclosure after taking heat treatment;
FIG. 2d is a cross-section SEM image of Specimen No. 11 of this disclosure after taking heat treatment;
FIG. 3 shows a chart comparing the cross-section SEM images and the copper concentrations of Specimen No. 1 and Specimen No. 11 of this disclosure after taking heat treatment;
FIG. 4 shows a chart comparing the cross-section SEM images and the copper concentration distributions of Specimens No. 2, No. 3, No. 4 and No. 5 of this disclosure having different thickness of the infiltration promoter layer;
FIG. 5 shows a chart comparing the cross-section SEM images and the copper concentration distributions of Specimens No. 6, No. 7, and No. 8 of this disclosure at different heat treatment temperature;
FIG. 6 shows a chart comparing the cross-section SEM images and the copper concentration distributions of Specimens No. 8, No. 9 and No. 10 of this disclosure at different heat treatment temperature maintaining time;

FIG. 7 shows a chart comparing the cross-section SEM images and the surface SEM image at the position of the diffusion depth of Specimen No. 8 of this disclosure after taking heat treatment; and

FIG. 8 is a chart showing and comparing the antibacterial test results of the stainless steel with the antibacterial alloy layer manufactured in accordance with this disclosure and a general stainless steel, wherein this disclosure uses a sterile test solution.

## DESCRIPTION OF THE EMBODIMENTS

This disclosure will now be described in more detail with reference to the accompanying drawings that show various embodiments of this disclosure. It is intended that the embodiments and drawings disclosed herein are to be considered illustrative rather than restrictive.

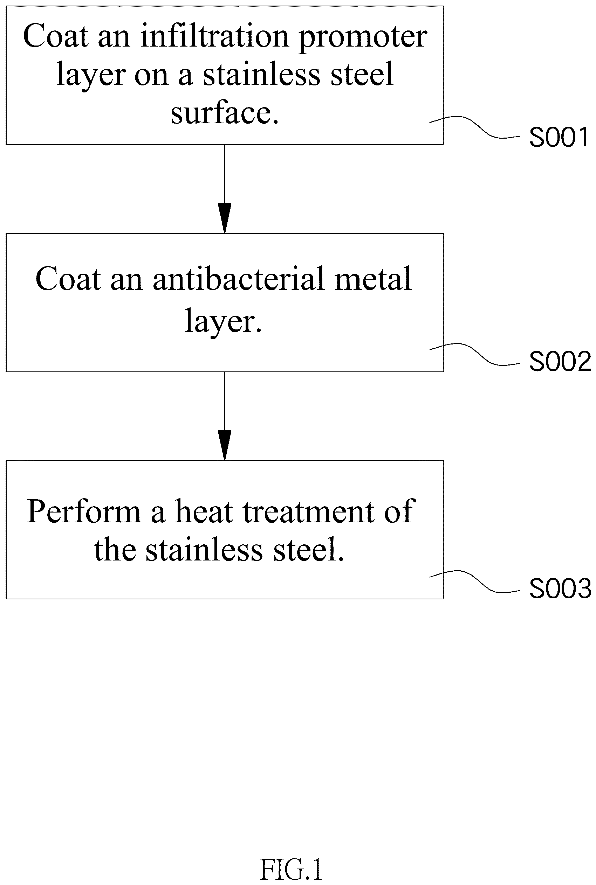

With reference to FIG. 1 for a method for preparing antibacterial stainless steel by surface alloying in accordance with this disclosure, the method includes the following steps:

S001: Coat an infiltration promoter layer on a stainless steel surface. In an embodiment, the infiltration promoter layer is made of silver, cobalt, nickel, zinc, molybdenum, or any combination of the above. In a specific embodiment, the stainless steel is at least one of the ferritic stainless steel, austenitic stainless steel or martensitic stainless steel. In an embodiment, the antibacterial metal layer is coated on the stainless steel by at least one method of the physical vapor deposition, chemical vapor deposition, electroplating or autocatalytic plating. However, the description above is provided for illustrating this disclosure only, but not intended for limiting the scope of this disclosure. In addition, the stainless steel serving as a base material can be of any structure or appearance such as a board, a plate, a semi-finished product, etc. It is noteworthy that whether or not the structure of the base material will cause an uneven diffusion of the infiltration promoter layer or the antibacterial metal coating.

As to the infiltration promoter layer, it can have any thickness, but preferably from 0.05 $\mu$m to 5 $\mu$m. If the thickness of the infiltration promoter layer is insufficient, then the effect of promoting the diffusion will be poor; and if the thickness of the infiltration promoter layer exceeds 5 $\mu$m, then the subsequent diffusion of the antibacterial metal will be hindered.

S002: Coat an antibacterial metal layer on the surface of the infiltration promoter layer. Wherein, the antibacterial metal of the antibacterial metal is copper. In another embodiment, the antibacterial metal includes infiltration promoting elements such as silver, cobalt, nickel, zinc, molybdenum, or any combination of the above, and the content of the infiltration promoter is less than the content of the antibacterial metal, and whether or not to add the infiltration promoter and the added content are determined according to the material of the stainless steel. When the antibacterial metal layer is coated on the stainless steel surface, the thickness can be any value, preferably from 0.1 $\mu$m to 5 $\mu$m. If the thickness of the antibacterial metal layer is less than 0.1 $\mu$m, it will be difficult to form an alloy layer with sufficient concentration on the stainless steel surface, so that it cannot guarantee a good antibacterial effect. If the thickness of the antibacterial metal layer exceeds 5 $\mu$m, the extra antibacterial layer will exist in the form of residual copper on the stainless steel surface, and it will be necessary to remove this precursor by surface treatment.

S003: Perform heat treatment of the stainless steel having the antibacterial metal layer. In an embodiment, the heat treatment heats in a protective atmosphere to a temperature above 600 °C, and the heating temperature is set to a range from 600°C to 1200°C, and the heating time is over one minute. In a specific embodiment, the protective atmosphere is carried out in an oxygen-free environment, a vacuum environment, or a protective gas (at least one selected from nitrogen, argon, and hydrogen), and the heating process can take place at any heating rate as needed. In this way, the antibacterial metal can be diffused into the stainless steel, and an antibacterial alloy layer with a specific thickness is formed at the surface and interior of the stainless steel.

In an embodiment, the heating temperature is set with a maintaining temperature at 600 °C, and can be adjusted according to the type, thickness, form and structure of the stainless steel. If the heating temperature is lower than 600 °C, it will be unable to generate sufficient driving force to drive the diffusion of the antibacterial metal. As the heating temperature increases, the diffusion rate will increase, which can effectively increase the diffusion distance. However, the melting point of copper is 1085 °C, and if the heating exceeds such temperature, too much copper will volatilize into the environment and cause furnace pollution, or it will be more likely that the sample sticks to the furnace body or other workpieces. Therefore, the heat treatment maintaining temperature preferably falls within a range of 1000 °C-1085 °C.

The heat treatment time is a least one minute to ensure that there is sufficient time to diffuse the antibacterial element into the substrate, and the length of the time can be adjusted according to the type of steel and the target thickness of the antibacterial alloy layer. When the heat treatment time increases, the diffusion distance of the antibacterial element will increase, so that the antibacterial alloy layer is thickened. On the other hand, if the heat treatment time is too long, it will increase the fusion and dilution of the antibacterial element in the stainless steel substrate, and thus causing a waste of the antibacterial metal and energy during the coating process. Therefore, the heat treatment time preferably falls within a range from one minute to 100 hours.

S004: Cool the stainless steel material after its diffusion and heat treatment to room temperature by using any cooling rate or method according to the target tissue structure. In this embodiment, the cooling treatment of the stainless steel having the antibacterial metal layer is carried out by at least one method selected from furnace cooling, air cooling or quench, and the stainless steel with the antibacterial metal layer is finally cooled to room temperature, so as to promote part of the copper element molten in stainless steel to be precipitated.

In the method of this disclosure, when the antibacterial metal layer is copper, the copper concentration will gradually decrease from the stainless steel surface along with diffusion. In the heat treatment process, the copper element will preferentially pass through the grain boundary with a faster diffusion rate to enter into the stainless steel, so that there will be an obvious enrichment of copper element at the grain boundary near the surface, and the copper element will be further diffused into the parent phase of the stainless steel and molten into the grains. Since the room-temperature copper has a lower saturation solubility compared with its high-temperature state, therefore during the cooling process, a part of the copper element originally molten in the grains will be segregated to form a copper-rich phase. Since there is an enrichment of the copper element at the grin boundary, the copper-rich phase will be produced more easily. Therefore, when the grain boundary on the stainless steel surface and the interior of the base material grains have high-density copper-rich phase tissues, the depth will increase, and the copper-rich tissue will decrease, and the concentration inside the grains of the copper element also gradually decrease to a level close to its original chemical composition.

As to the thickness of the antibacterial alloy layer, when the copper element molten in the stainless steel has reached 3wt% or above, the antibacterial effect can be achieved. Therefore, in this disclosure, the diffusion depth or the thickness of the antibacterial alloy layer is the distance measured inward to the position where the melting quantity of the copper content in the substrate is 3 wt.% by using the stainless steel surface as the benchmark, and this benchmark does onto include the copper plating precursor remained after the diffusion, In this disclosure, the thickness of the antibacterial alloy layer is measured by a scanning electron microscope (SEM) to observe the microscopic tissue structure. At the same time, an energy dispersive spectrometer (EDS) is used for element analysis and measure the copper element diffusion depth. In order to improve the precision of the quantitative measurement, and EDS adopts point measurement and performs vertical analysis at several different positions to determine the thickness of the antibacterial alloy layer

In this disclosure, several embodiments are used to further elaborate the technical characteristics of this disclosure. It is noteworthy that these embodiments are used as exemplary illustration, but not intended for limiting the scope of this disclosure.

In an experimental example of this disclosure, the 440C stainless steel (SS440C) is selected and used as the stainless steel of this disclosure, and its chemical composition is shown in Table 1, and the following method is used for pre-treatment: First, a stainless steel bar is cut into a size of 20 mm (length), 12 mm (width), and 0.2 mm (thickness), and then a silicon carbide sandpaper or any other abrasive material is used to grind and remove surface rust or foreign substances, such that the surface has an appropriate roughness, and then deionized water is used to rinse the test specimen, and ultrasonic shock is used to clean the test specimen surface after the rinsing of the test specimen; and the test specimen is put into a plating solution for electroplating, and the deionized water is used to rinse the test specimen surface, and a diffusion heat treatment is performed. The diffusion heat treatment is conducted in a protective gas, and the heat treatment temperature falls within a range of 800°C-1080°C, and the heat treatment time is one to 36 hours, and finally the specimen is cooled to room temperature.

【Table 1】

| Element⟍Material | C | Mn | P | S | Si | Cr | Mo | Fe |
|---|---|---|---|---|---|---|---|---|
| SS440C | 0.95-1.20 | <1.00 | <0.04 | <0.03 | <1.00 | 16.00-18.00 | <0.75 | Bal. |

(Unit: wt.%)

Processing parameters of different types of stainless steel used in the embodiments of this disclosure are listed in Table 2 below.

[Table 2]

| Specimen No. | Type of Steel | Thickness of Infiltration Promoter (um) | Electroplating Time Copper (min) | Heat Treatment | | Remarks |
|---|---|---|---|---|---|---|
| | | | | Maintaining Temperature (°C) | Temperature Maintaining Time (hr) | |
| 1 | SS440C | 1.5 | 10 | 1000 | 5 | Experimental examples of this disclosure |
| 2 | SS440C | 1 | 10 | 1000 | 24 | |
| 3 | SS440C | 1.4 | 10 | 1000 | 24 | |
| 4 | SS440C | 1.9 | 10 | 1000 | 24 | |
| 5 | SS440C | 2.8 | 10 | 1000 | 24 | |
| 6 | SS440C | 1.4 | 10 | 800 | 12 | |
| 7 | SS440C | 1.3 | 10 | 1000 | 12 | |
| 8 | SS440C | 1.7 | 10 | 1080 | 12 | |
| 9 | SS440C | 1.8 | 10 | 1080 | 24 | |
| 10 | SS440C | 1.7 | 10 | 1080 | 36 | |
| 11 | SS440C | 0 | 10 | 1000 | 5 | Control |

[Influence of Infiltration Promoter Layer]

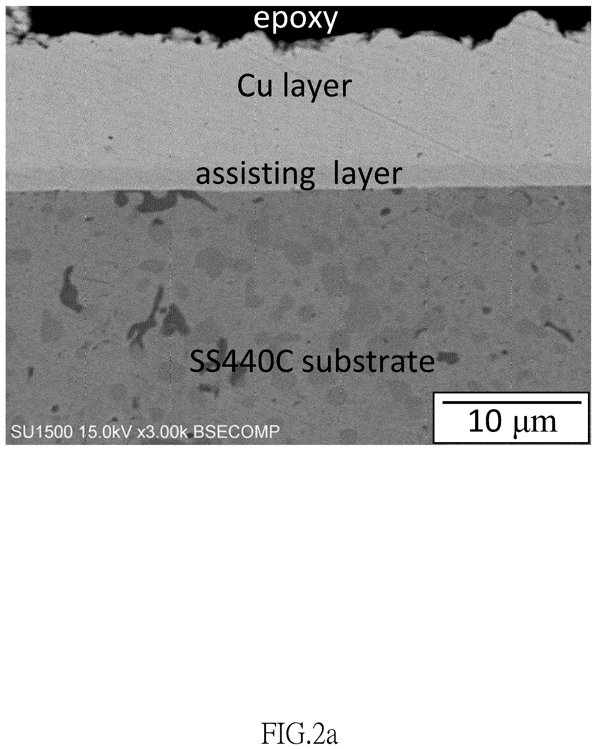

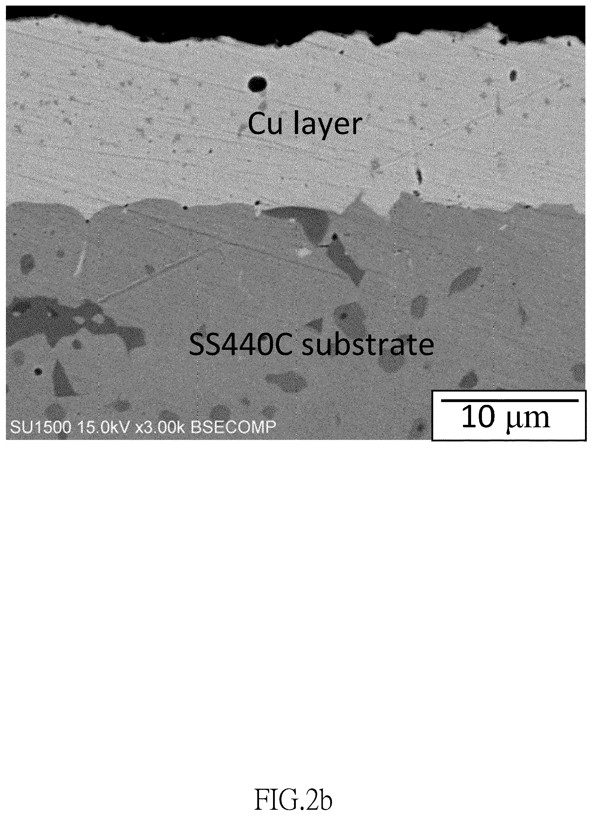

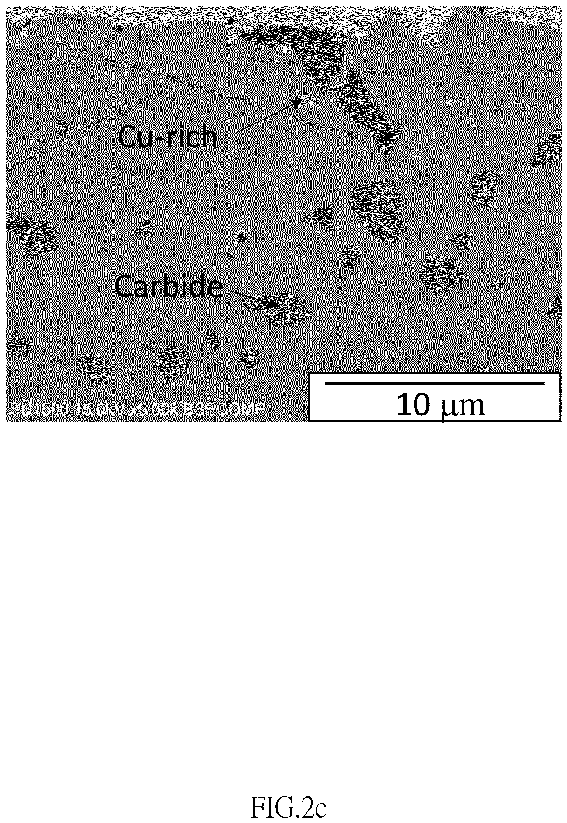

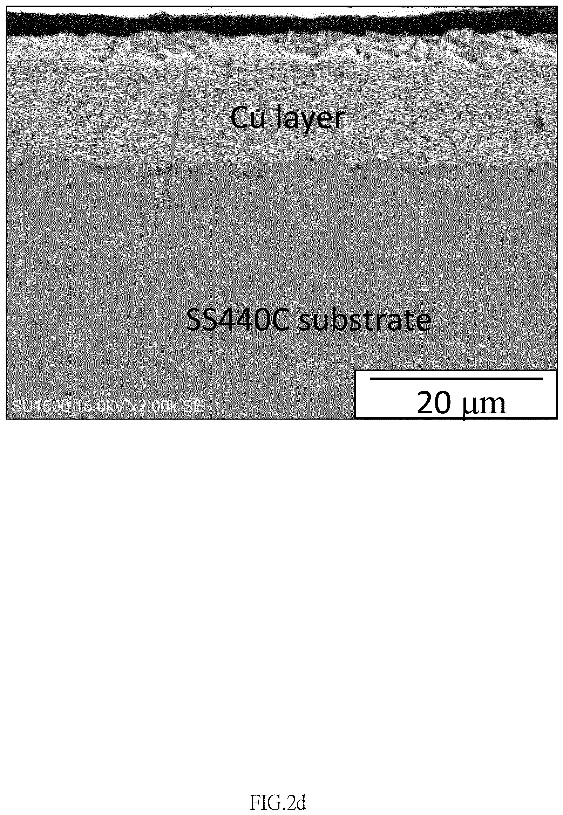

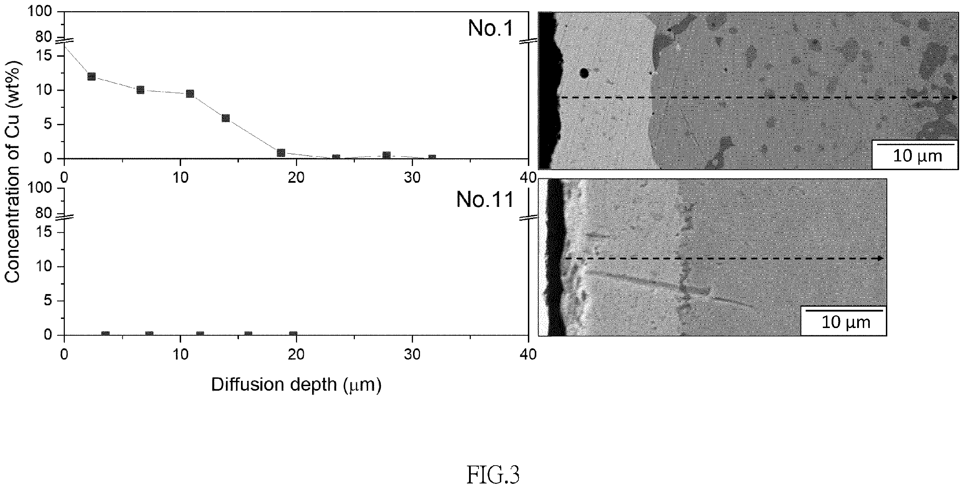

With reference to FIG. 2a for the cross-section microscopic tissue of the Specimen No. 1 after the electroplating, the infiltration promoter layer is sandwiched between the stainless steel substrate and the copper layer. After the heat treatment, a copper layer is obviously remained on the stainless steel substrate surface as shown in FIG. 2b. Fig. 2c shows an amplified image of the stainless steel substrate surface with the residual copper precursor, and it is obviously found that copper-rich phase is formed. On the contrary, the Specimen No. 11 without being processed with the diffusion infiltration promoter is provided as a control of this experimental example for comparison. After the heat treatment, there is no copper-rich phase formed in the substrate of this specimen as shown in FIG. 2d; and the gradient analysis results on the EDS composition of the two Specimens No. 1 and No. 11 are compared as shown in FIG. 3, it is found that in the experimental example of Specimen No. 1 has an obvious copper diffusion effect with a diffusion distance up to 20 $\mu$m; and no copper is diffused or molten into the stainless steel for the Specimen No. 11 without the infiltration promoter layer.

[Influence on the Thickness of the Infiltration Promoter Layer]

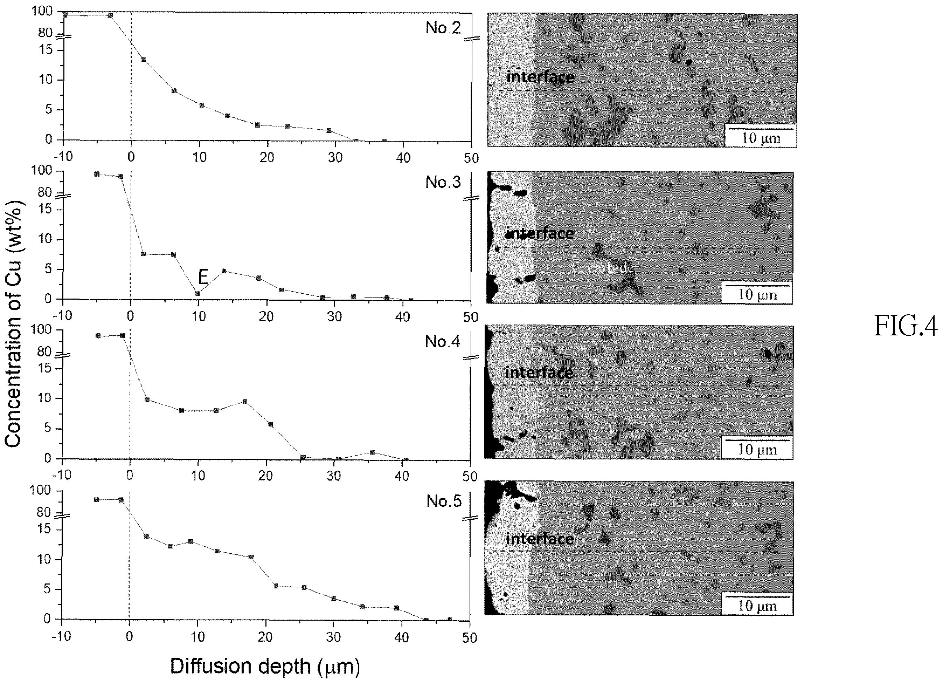

With reference to FIG. 4 for the comparison of the influence of different thickness of the infiltration promoter layer on the copper diffusion, Specimens No. 2, No. 3, No. 4 and No. 5 are provided with different thickness of the infiltration promoter layer, and the copper electroplating and diffusion heat treatment are conducted, and EDS is used to analyse the gradient change of the copper element composition. The analysis result shows that the copper diffusion depth is 17.5 $\mu$m for Specimen No. 2, 26.7 $\mu$m for Specimen No. 3, 30.0 $\mu$m for Specimen No. 4, and 32.1 $\mu$m for Specimen No. 5, which indicates that the diffusion depth in the stainless steel base material increases with the thickness of the infiltration promoter layer, and the copper also increases, and the copper concentration of the copper molten in the stainless steel near the surface also increases. The foregoing results show that the use of the diffusion infiltration promoter facilitates the copper to be molten into the stainless steel surface to give an obvious alloying effect.

[Influence on Heat Treatment Temperature]

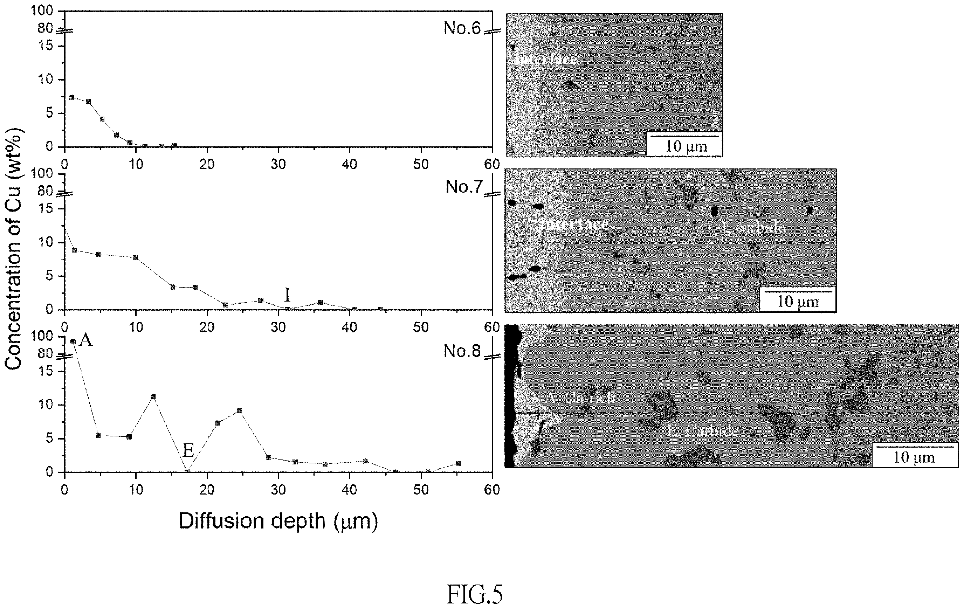

With reference to FIG. 5 for the copper concentration distributions at different heat treatment temperature; the Specimens No. 6, No. 7 and No. 8 have a heat treatment carried out at 800 °C, 1000 °C and 1080 °C for a temperature maintaining time of 12 hours respectively, the gradient analysis results of the copper element concentration show that the copper concentration uses 3 wt.% as a benchmark, the copper diffusion depths are 6 $\mu$m (for Specimen No. 6), 18 $\mu$m (for Specimen No. 7) and 25 $\mu$m (for Specimen No. 8); and these results show that an increase of the heat treatment temperature can improve the copper diffusion depth, and increase the thickness of the antibacterial alloy layer.

[Influence on Heat Treatment Time]

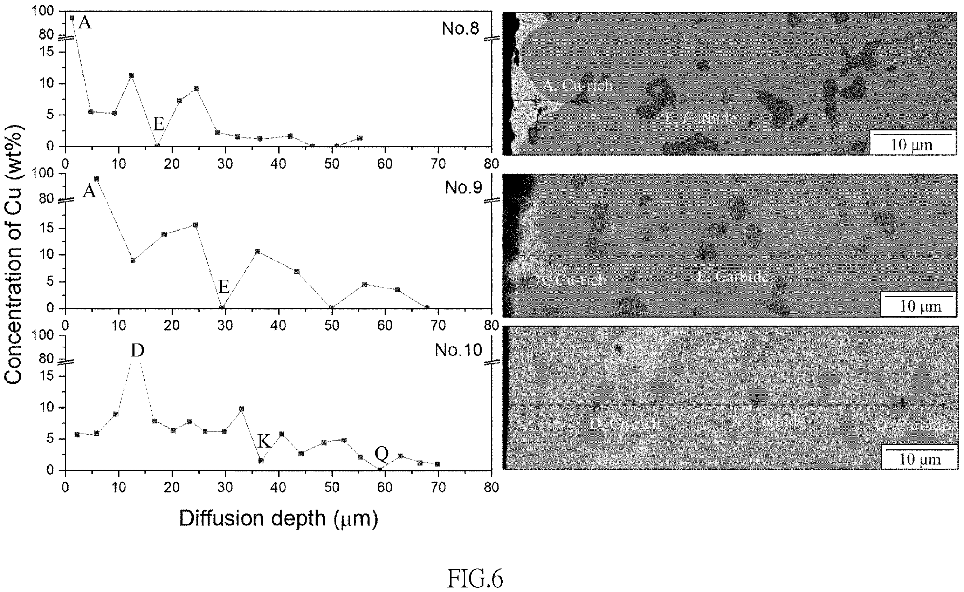

With reference to FIG. 6 for the influence of the heat treatment temperature maintaining time on the distribution of the concentration of the copper element molten in the stainless steel at a constant temperature, the heat treatment of Specimens No. 8, No. 9 and No. 10 are conducted under a constant temperature at 1080 °C for 12 hours, 24 hours and 36 hours respectively, and then the copper concentration of 3 wt.% is used as a benchmark, and the copper element diffusion depths are 25 $\mu$m, 55 $\mu$m and 60 $\mu$m respectively. These results show that increasing the heat treatment time at a constant temperature can promote the diffusion of the copper element and increase the thickness of the antibacterial alloy layer.

[Organization Form of Different Depth Planes]

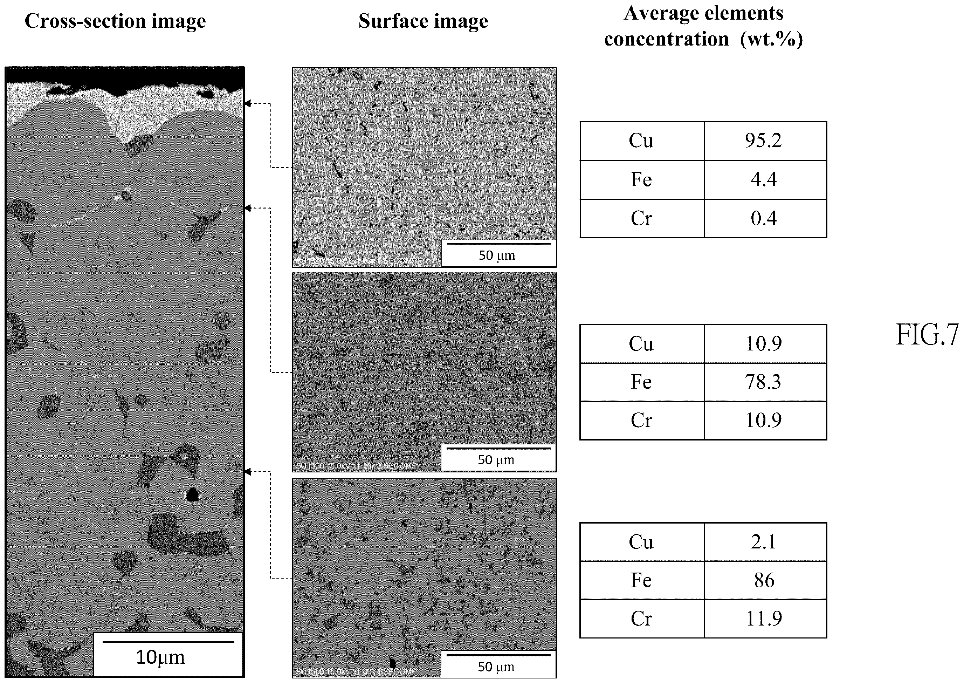

With reference to the left of FIG. 7 for the cross-section image of Specimen No. 8 closely attached to the bottom of the copper precursor near the surface, and the right of FIG. 7 for the images (which are captured in a direction perpendicular to the copper diffusion direction) of the surfaces at the position of different copper element diffusion depths; the SEM/EDS analysis results show that the copper concentration surely decreases with the increase of the diffusion distance, and when the copper concentration drops to approximately 2 wt.%, the existence of copper precipitates is still found on the SEM images.

[Antibacterial Test]

The antibacterial test adopts the DH5$\alpha$ Escherichia coli as the test strain, and the bacterial liquid with a concentration of $2.5 \times 10^5 \sim 10^6$ CFU/ml is inoculated on antibacterial-treated and untreated stainless steel test specimens, with a control test area of 15 mm $\times$ 10 mm, and incubated at 37°C for 24 hours; the bacterial solution was subsequently eluted with phosphate buffered saline (PBS), and then the standard plate count method is used to calculate the colony count, wherein the colony count of the two test specimens is calculated according to the following formula 1 to obtain the antibacterial rate of the antibacterial treated test specimen.

[Mathematical Equation 1]

$$\text{Antibacterial Rate} = (\text{Colony Count of Untreated Stainless Steel Test Specimen - Colony Count of Treated Stainless Steel Test Specimen})/\text{Colony Count of Untreated Stainless Steel Test Specimen}) \times 100\%$$

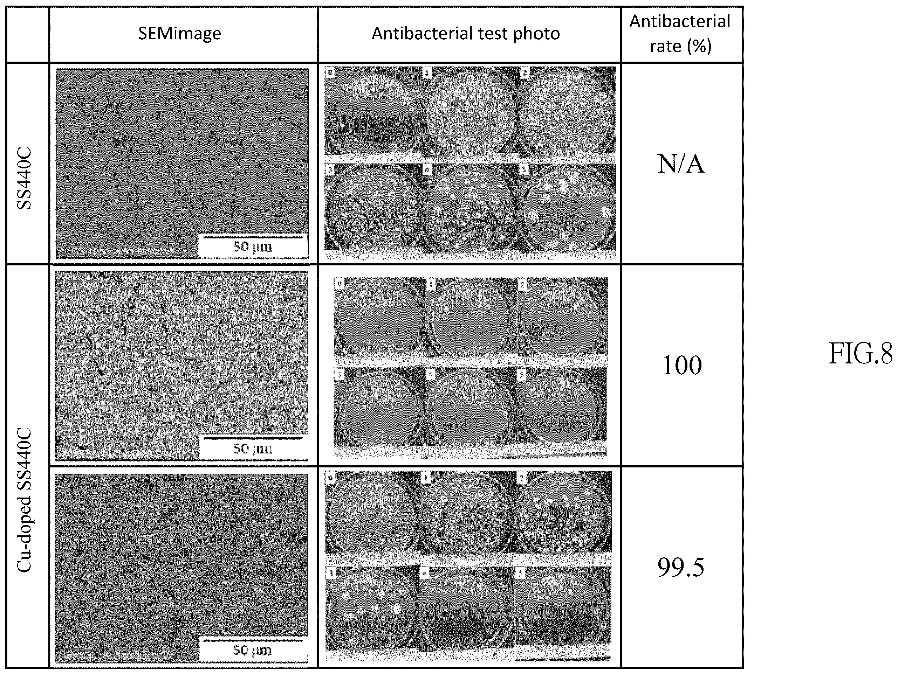

The results of the aforementioned antibacterial test of the copper-bearing stainless steel formed by surface alloying (as shown in Fig. 8) shows that the copper-bearing stainless steel formed by surface alloying has excellent antibacterial effect in this experimental example, thereby confirming that the antibacterial alloy layer can be effectively formed on the stainless steel surface by the method of this disclosure

**Claims**

1. A method for preparing antibacterial stainless steel by surface alloying, comprising the steps of:

   coating an infiltration promoter layer on a stainless steel surface;
   coating an antibacterial metal layer on a surface of the infiltration promoter layer; and
   performing a heat treatment of the stainless steel to diffuse the antibacterial metal into the stainless steel.

2. The method for preparing antibacterial stainless steel by surface alloying according to claim 1, wherein the stainless steel is at least one selected from the group consisting of ferritic stainless steel, austenitic stainless steel, martensitic stainless steel, and duplex stainless steel.

3. The method for preparing antibacterial stainless steel by surface alloying according to claim 1, wherein the antibacterial metal layer is coated onto the stainless steel surface by at least one method selected from the group consisting of physical vapor deposition, chemical vapor deposition, electroplating, and autocatalytic plating.

4. The method for preparing antibacterial stainless steel by surface alloying according to claim 1, wherein the antibacterial metal layer is coated when the thickness of the stainless steel surface falls within a range from 0.1μm to 15μm.

5. The method for preparing antibacterial stainless steel by surface alloying according to claim 1, wherein the heat treatment heats the protective atmosphere up to a temperature above 600°C for a heating time of more than 1 minute.

6. The method for preparing antibacterial stainless steel by surface alloying according to claim 5, further comprising the step of performing a cooling treatment for the stainless steel with the antibacterial metal layer by at least one method selected from the group consisting of furnace cooling, air cooling) and quench.

7. The method for preparing antibacterial stainless steel by surface alloying according to claim 1, wherein the cooling treatment cools the stainless steel with the antibacterial metal layer to room temperature.

8. The method for preparing antibacterial stainless steel by surface alloying according to claim 1, wherein the antibacterial metal is copper.

9. The method for preparing antibacterial stainless steel by surface alloying according to claim 1, wherein the infiltration promoter layer is one selected from the group consisting of silver, cobalt, nickel, zinc, and any combination thereof.

10. The method for preparing antibacterial stainless steel by surface alloying according to claim 9, wherein the infiltration promoter element has a content less than the content of the antibacterial metal.

Coat an infiltration promoter layer on a stainless steel surface. — S001

Coat an antibacterial metal layer. — S002

Perform a heat treatment of the stainless steel. — S003

FIG.1

FIG.2a

FIG.2b

FIG.2c

Cu layer

SS440C substrate

SU1500 15.0kV x2.00k SE

20 μm

FIG.2d

No.1

No.11

Concentration of Cu (wt%)

Diffusion depth (μm)

10 μm

10 μm

FIG.3

FIG.4

EP 4 596 761 A1

FIG.5

EP 4 596 761 A1

FIG.6

EP 4 596 761 A1

Cross-section image

Surface image

Average elements concentration (wt.%)

| Cu | 95.2 |
|----|------|
| Fe | 4.4 |
| Cr | 0.4 |

| Cu | 10.9 |
|----|------|
| Fe | 78.3 |
| Cr | 10.9 |

| Cu | 2.1 |
|----|------|
| Fe | 86 |
| Cr | 11.9 |

FIG.7

| | SEMimage | Antibacterial test photo | Antibacterial rate (%) |
|---|---|---|---|
| SS440C | | | N/A |
| Cu-doped SS440C | | | 100 |
| | | | 99.5 |

FIG.8

Europäisches
Patentamt
European
Patent Office
Office européen
des brevets

# EUROPEAN SEARCH REPORT

**Application Number**

EP 24 15 4622

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | JP H11 106987 A (DAIDO STEEL CO LTD) 20 April 1999 (1999-04-20) | 1,3-5, 7-10 | INV. C25D5/36 |
| Y | * paragraphs [0004] - [0007] * | 6 | C25D5/50 C23C14/16 |
| X | JP H11 193495 A (DAIDO STEEL CO LTD) 21 July 1999 (1999-07-21) | 1-5,7-9 | C23C14/58 C23C16/06 |
| Y | * paragraphs [0016] - [0018], [0022] * | 6 | |
| X | JP 2005 133190 A (KANAI HIROAKI) 26 May 2005 (2005-05-26) * paragraph [0012] * | 1,3,4,9 | ADD. C25D3/38 |
| X | CN 101 880 860 A (UNIV TAIYUAN TECHNOLOGY) 10 November 2010 (2010-11-10) | 1,3,10 | |
| Y | * Summary of the invention * * claim 1 * | 6 | |

**TECHNICAL FIELDS SEARCHED (IPC)**

C25D
C23C

The present search report has been drawn up for all claims

| Place of search | Date of completion of the search | Examiner |
|---|---|---|
| The Hague | 10 July 2024 | Le Hervet, Morgan |

CATEGORY OF CITED DOCUMENTS

X : particularly relevant if taken alone
Y : particularly relevant if combined with another
    document of the same category
A : technological background
O : non-written disclosure
P : intermediate document

T : theory or principle underlying the invention
E : earlier patent document, but published on, or
    after the filing date
D : document cited in the application
L : document cited for other reasons
.............................................................................................
& : member of the same patent family, corresponding
    document

EPO FORM 1503 03.82 (P04C01)

## ANNEX TO THE EUROPEAN SEARCH REPORT
## ON EUROPEAN PATENT APPLICATION NO.

EP 24 15 4622

This annex lists the patent family members relating to the patent documents cited in the above-mentioned European search report.
The members are as contained in the European Patent Office EDP file on
The European Patent Office is in no way liable for these particulars which are merely given for the purpose of information.

10-07-2024

| Patent document cited in search report | Publication date | Patent family member(s) | Publication date |
|---|---|---|---|
| JP H11106987 A | 20-04-1999 | NONE | |
| JP H11193495 A | 21-07-1999 | NONE | |
| JP 2005133190 A | 26-05-2005 | NONE | |
| CN 101880860 A | 10-11-2010 | NONE | |

EPO FORM P0459

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 1764530 A